Europäisches Patentamt

European Patent Office

Office européen des brevets

(19)

(11) Veröffentlichungsnummer: **0 025 814**
**B1**

(12) **EUROPÄISCHE PATENTSCHRIFT**

(45) Veröffentlichungstag der Patentschrift: **02.05.84**

(51) Int. Cl.³: **A 61 F 1/03**

(21) Anmeldenummer: **80100850.9**

(22) Anmeldetag: **21.02.80**

(54) Einteiliger Oberschenkelteil einer Hüftgelenkendoprothese.

(30) Priorität: **04.08.79 DE 2931750**

(43) Veröffentlichungstag der Anmeldung:
**01.04.81 Patentblatt 81/13**

(45) Bekanntmachung des Hinweises auf die Patenterteilung:
**02.05.84 Patentblatt 84/18**

(84) Benannte Vertragsstaaten:
**AT BE CH FR GB IT LU NL SE**

(56) Entgegenhaltungen:
**DE - A - 1 491 246**
**DE - A - 1 943 598**
**DE - B - 2 517 702**
**DE - U - 1 949 841**
**FR - A - 2 021 313**

(73) Patentinhaber: **Howmedica International, Inc.**
**Zweigniederlassung Kiel**
**Professor-Küntscher-Strasse 1-5**
**D-2301 Schönkirchen üb. Kiel (DE)**

(72) Erfinder: **Seidel, Hartmut, Dr. med.**
**Hochrad 19**
**D-2000 Hamburg 52 (DE)**
Erfinder: **Richter, Karl M., Dipl.-Ing.**
**Haferkamp 14**
**D-2304 Wendtorf (DE)**
Erfinder: **Harder, Hans Erich**
**Mecklenburger Strasse 32**
**D-2301 Probsteierhagen (DE)**
Erfinder: **Behrens, Klaus, Ing.grad.**
**Am Sportplatz 8**
**D-2351 Rickling (DE)**

(74) Vertreter: **Hauck, Hans, Dipl.-Ing. et al,**
**Patentanwälte Dipl.-Ing.H.Hauck, Dipl.-**
**Phys.W.Schmitz, Dipl.-Ing.E.Graalfs, Dipl.-**
**Ing.W.Wehnert, Dipl.-Phys.W.Carstens Dr.-**
**Ing.W.Döring Neuer Wall 41**
**D-2000 Hamburg 36 (DE)**

Courier Press, Leamington Spa, England.

Einteiliger Oberschenkelteil einer Hüftgelenkendoprothese

Die Erfindung bezieht sich auf einen einteiligen Oberschenkelteil einer Hüftgelenkendoprothese mit einem Kopf, einem in der Frontalebene konkav bzw. konvex gekrümmte Seiten aufweisenden und sich in der Frontalebene stetig nach unten verjüngenden, mit Knochenzement im Femur verankerbaren Schaft und einem zwischen Schaft und Kopf angeordneten Halsabschnitt.

Ein derartiger Oberschenkelteil ist vielfach bekannt geworden (z.B. DE—A—27 47 867). Der Schaft wird mit Hilfe von Knochenzement in der Markhöhle des Femurs verankert, und der Gelenkkopf wirkt mit der Hüftpfanne im Beckenknochen zusammen oder mit einer aus Metall oder Kunststoff bestehenden Hüftpfanne, welche die Knorpelschicht des Acetabulums ersetzt. Der sich in der Frontalebene stetig nach unten verjüngende Schaft ist bananenförmig gekrümmt, wobei die laterale Seite eine konvexe Krümmung besitzt.

Hüftgelenkendoprothesen sind naturgemäß erheblichen statischen und dynamischen Belastungen ausgesetzt, so daß trotz erheblicher Fortschritte sowohl in der Ausbildung der Endoprothesen als auch in der medizinischen Versorgung die Gefahr noch nicht gebannt ist, daß sich die Prothesenteile mit der Zeit lockern, wobei die innige Verbindung zwischen Prothese und Knochen, welche durch den Knochenzement bewirkt werden soll, allmählich verloren geht.

Aus einer zeichnerischen Darstellung der DE—U—1 949 841 ist ein Oberschenkelteil für Hüftgelenkendoprothesen bekannt, bei dem die mediale Seite des Schaftes konvex und die laterale Seite des Schaftes konkav gekrümmt ist. Ob der Oberschenkelteil ohne oder mit Knochenzement implantiert wird und wie der Oberschenkelteil in der Markhöhle des Femurs plaziert ist, ist nicht offenbart.

Der Erfindung liegt der Aufgabe zugrunde, einen Oberschenkelteil einer Hüftgelenkendoprothese zu schaffen, welches mit Hilfe von Knochenzement mit größtmöglicher Sicherheit in der Markhöhle des Femurs verankert werden kann bei bestmöglicher Übertragung dynamischer und statischer Kräfte und größtmöglicher Haltbarkeit der Verbindung.

Diese Aufgabe wird erfindungsgemäß dadurch gelöst, daß die sich in Schaftlängsrichtung erstreckende mediale Seite des Schaftes konvex und die sich in Schaftlängsrichtung erstreckende laterale Seite konkav gekrümmt ist, wobei die Krümmung so gewählt ist, daß der mittlere Bereich des Schaftes im implantierten Zustand an der medialen Corticalis des Femurs anliegt und wobei der untere Endbereich des Schaftes so geformt ist, daß er sich im implantierten Zustand an der lateralen Corticalis abstützt.

Die beim erfindungsgemäßen Oberschenkelteil gewählte Schaftkrümmung ermöglicht eine breite Zementfüllung am Trochanter minor und eine optimale Ausnutzung des Trochanter major. Die Krümmung wirkt außerdem wie eine innere Zuggurtung, welche sich außerordentlich günstig hinsichtlich auftretender Zug- und Biegebeanspruchung auswirkt. Dieser Umstand ist insofern von großer Bedeutung, als vorübergehende Durchbiegungen des Prothesenschaftes infolge von Belastungsstößen sehr leicht eine Lockerung des Verbundes mit dem Knochenzement zur Folge haben.

Eine sichere und haltbare Verankerung im Femur wird vor allem auch dadurch erzielt, daß das Oberschenkelteil sich doppelt abstützt an gegenüberliegenden Seiten des Schaftes. Eine derartige Abstützung widersetzt sich einer Formänderung, so daß die Gefahr einer Lockerung stark vermindert wird. Zu diesem Zweck sieht eine weitere Ausgestaltung der Erfindung vor, daß der untere Endbereich eine annähernd parallel zur Wandung der Markhöhle im Bereich der lateralen Corticalis verlaufende laterale Anlagefläche aufweist.

Eine andere Ausgestaltung der Erfindung sieht vor, daß die Breite des Schaftes in der Frontalebene im oberen und mittleren Bereich größer ist als senkrecht dazu, vorzugsweise annähernd eineinhalb bis zweifach so groß. Dadurch wird eine breitflächige Abstützung des Oberschenkelteils im Knochenzement erreicht und verhindert, daß der Zement sich spaltet.

Eine weitere Ausgestaltung der Erfindung sieht vor, daß die in der Frontalebene liegende Vorder- und/oder Rückseite des Schaftes mehrere Ausnehmungen aufweist. Vorder- und/oder Rückseite des Schaftes sind in einer weiteren Ausgestaltung der Erfindung hierzu wabenartig ausgebildet. Dadurch wird eine noch breitflächigere Abstützung des Oberschenkelteils im Zement erzielt und die Ableitung von Polymerisationswärme bei der Abbindung des Knochenzementes verbessert.

Eine weitere Ausgestaltung der Erfindung sieht vor, daß der Halsabschnitt zum Schaft hin in einen sich allmählich verbreiternden Kragen übergeht, der an der Unterseite eine Schulterfläche zur Auflage auf das proximale Ende des bearbeiteten Femurs bildet. Auf diese Weise ist eine Auflage geschaffen, die unter Belastung dem knochensprengenden Keileffekt des Oberteils entgegenwirkt. Im übrigen wird die Zug- und Biegebeanspruchung des Schaftes durch die Auflage auf den Knochen verringert. Die Ausbildung eines derartigen Kragens ist jedoch an sich bekannt (DE—A—2 747 867). Sie ist jedoch in Verbindung mit dem erfindungsgemäßen Oberschenkelteil von besonderem Vorteil.

Beim Einschlagen herkömmlicher Oberschenkelteile besteht die Gefahr, daß der Gelenkkopf beschädigt wird. Daher sieht eine weitere Ausgestaltung der Erfindung vor, daß an der lateralen Seite des Halsabschnitts ein Einschlagansatz angeformt ist. Mit Hilfe eines geeigneten Instruments kann das Oberschenkelteil ohne Beschädigung des Kopfes fest eingeschlagen werden. Der Einschlagansatz kann ferner zu Extraktionszwecken verwendet werden, wenn er gemäß einer weiteren Ausgestaltung der Erfindung so geformt ist, daß er eine Öffnung oder einen Hinterschnitt oder dergleichen aufweist zwecks Zusammenwirkung mit einem Extraktionswerkzeug.

Ein Ausführungsbeispiel der Erfindung wird nachfolgend anhand von Zeichnungen näher erläutert.

Fig. 1 zeigt der Vorderansicht eines Oberschenkelteils nach der Erfindung für den rechten Femur.

Fig. 2 zeigt die laterale Ansicht des Oberschenkelteils nach Fig. 1 teilweise im Schnitt.

Fig. 3 zeigt einen Schnitt durch das Oberschenkelteil nach Fig. 1 entlang der Linie III—III.

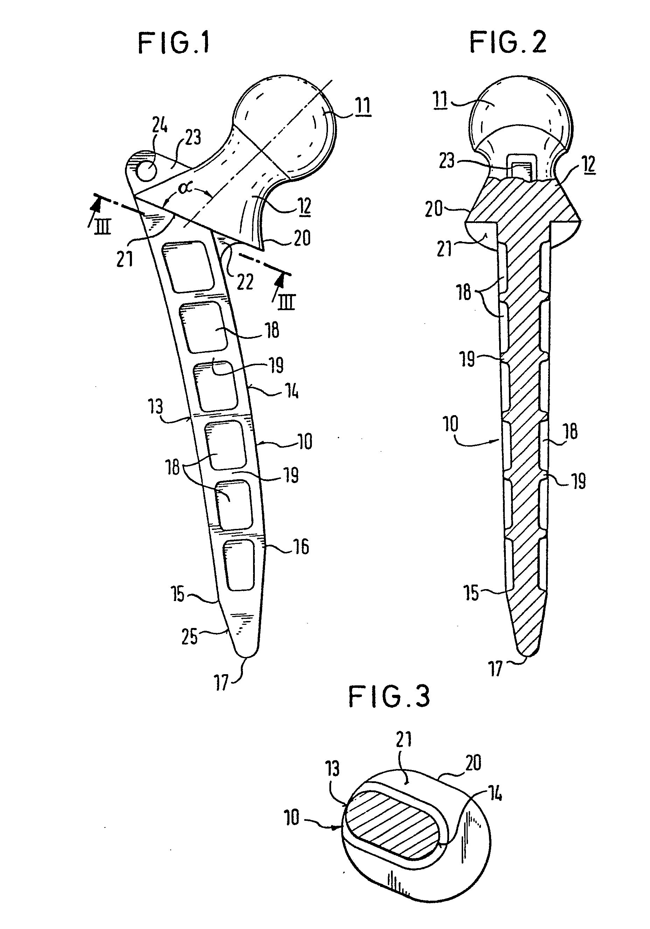

Der in den Figuren 1 bis 3 dargestellte Oberschenkelteil einer Hüftgelenkendoprothese für ein rechtes Hüftgelenk besitzt einen Schaft 10, einen kugeligen Gelenkkopf 11 und einen Halsabschnitt 12. Das einteilige Oberschenkelteil besteht aus Co Cr Mo-Material. Der Kopf 11 hat eine hochglanzpolierte Oberfläche, während der Rest matt ist. Der Kopfdurchmesser ist vorzugsweise 32 mm.

In der Frontalebene (Fig. 1) ist die laterale Seite 13 des Schaftes 10 konkav und die mediale Seite 14 konvex gekrümmt. Die Krümmung erfolgt vorzugsweise mit dem gleichen Radius, vorzugsweise 600 mm. Die konkave Krümmung der Seite 13 endet im unteren Bereich bei 15 und die konvexe Krümmung endet bei 16. Von den Punkten 15 und 16, wobei der letztere etwas höher liegt, verjüngt sich der Schaft 10 nach unten in der gezeigten Ansicht nach Fig. 1 dreieckförmig mit abgerundeter Spitze 17. Die in Fig. 1 linke Seite des unteren Endbereichs, die mit 25 bezeichnet ist, bildet eine Anlagefläche zur Anlage an die laterale Corticalis in der nicht gezeigten Femur-Markhöhle. Wie aus den Figuren 1 und 2 in Gesamtschau zu erkennen, bildet der untere Endbereich annähernd einen Kegelstumpf.

Aus Fig. 1 ist zu entnehmen, daß Vorder- und Rückseite des Schaftes 10 von oben nach unten bis zu den Punkten 15 und 16 sich etwas in der Breite verringern. Eine minimale Verjüngung ergibt sich auch in einer Ebene senkrecht dazu, wie aus Fig. 2 zu erkennen. Der Verjüngungswinkel gegenüber der Vertikalen beträgt jedoch lediglich um 1°.

Aus den Figuren 1 bis 3 ist ferner erkennbar, daß die Breite des Schaftes 10 in der Frontalebene größer ist als senkrecht dazu. Vorzugsweise beträgt die größere Breite das eineinhalbbis zweifache der kleineren Breite. Aus Fig. 3 ist zu entnehmen, daß Vorder- und Rückseite des Schaftes 10 parallel zueinander verlaufen und—senkrecht zur Frontalebenelaterale Seite 13 und mediale Seite 14 konvex gewölbt sind, vorzugsweise mit dem gleichen Radius.

Vorder- und Rückseite des Schaftes 10 besitzen jeweils eine Reihe von Ausnehmungen 18, die rechteckigen Umriß aufweisen und eine flache Bodenfläche, wobei die Größe der Ausnehmungen 18 von oben nach unten abnimmt. Die Ausnehmungen 18 verleihen dem Schaft eine wabenförmige Struktur. Die einzelnen Ausnehmungen sind durch Stege 19 voneinander getrennt, deren Oberkante in die laterale Seite bzw. mediale Seite 13 bzw. 14 des Schaftes übergeht, wie aus Fig. 2 zu erkennen.

Der Halsabschnitt 12 erweitert sich in Richtung Schaft 10 annähernd konisch und bildet somit einen Kragen 20, welcher an seiner Unterseite eine ebene Schulterfläche 21 bildet, die gegenüber der Achse des Halsabschnitts 12 bzw. des Kopfes 11 einen Winkel $\alpha$ von 113° einschließt. Dieser Winkel entspricht annähernd dem Winkel von Schenkelhals zu Femurachse bei einem älteren Menschen.

Der Schaft 10 geht in den Kragen 20 über einen konkav geformten Abschnitt 22 über.

Am Halsabschnitt 12 ist auf der lateralen Seite ein Ansatz 23 angeformt mit einer Durchbohrung 24. Auf der linken Seite (in Fig. 1) geht der Ansatz 23 glatt in die laterale Seite des Schaftes 10 über. Die obere Seite des Ansatzes 23 dient zum Einschlagen des dargestellten Oberschenkelteils, während das Loch 24 zu Extraktionszwecken herangezogen werden kann.

Das dargestellte Oberschenkelteil ermöglicht eine doppelte Flächenabstützung im Markraum, und zwar an der medialen Corticalis im Mittenbereich des Schaftes 10 und an der lateralen Corticalis an der Schaftspitze, nämlich mit der Fläche 25. Die aus Fig. 1 zu entnehmende Krümmung des Schaftes 10 ermöglicht eine breite Zementfüllung am Trochanter minor und eine optimale Ausnutzung des Trochanter major zur Verankerung. Die Wabenform verbessert die Abstützung der Prothese im Zement und verbessert die Ableitung von Polymerisationswärme. Die verhältnismäßig breite Vorder- und Rückfläche des Schaftes 10 verhindert eine Spaltung des Zements.

Die breite Prothesenauflage mit Hilfe der Schulterfläche 21 wirkt unter Belastung einem knochensprengenden Keileffekt des Oberschenkelteils entgegen und verringert die Zug- und Biegebeanspruchung des Schaftes.

**Patentansprüche**

1. Einteiliger Oberschenkelteil einer Hüftgelenkendoprothese mit einem Kopf (11), einem in der Frontalebene konkav bzw. konvex gekrümmte Seiten aufweisenden und sich in der Frontalebene stetig nach unten verjüngenden, mit Knochenzement im Femur verankerbaren Schaft (10) und einem zwischen Schaft (10) und Kopf (11) angeordneten Halsabschnitt (12), dadurch gekennzeichnet, daß die sich in Schaftlängsrichtung erstreckende mediale Seite (14) des Schaftes (10) konvex und die sich im Schaftlängsrichtung erstreckende laterale Seite (13) konkav gekrümmt ist, wobei die Krümmung so gewählt ist, daß der mittlere Bereich des Schaftes (10) im implantierten Zustand an der medialen Corticalis des Femurs anliegt und wobei der untere Endbereich (25) des Schaftes (10) so geformt ist, daß er sich im implantierten Zustand an der lateralen Corticalis abstützt.

2. Oberschenkelteil nach Anspruch 1, dadurch gekennzeichnet, daß der untere Endbereich eine annähernd parallel zur Wandung der Markhöhle im Bereich der lateralen Corticalis verlaufende laterale Anlagefläche (25) aufweist.

3. Oberschenkelteil nach Anspruch 1 oder 2, dadurch gekennzeichnet, daß die Breite des Schaftes (10) in der Frontalebene im oberen und mittleren Bereich größer ist als senkrecht dazu, vorzugsweise annähernd eineinhalb bis zweifach so groß.

4. Oberschenkelteil nach einem der Ansprüche 1 bis 3, dadurch gekennzeichnet, daß die in der Frontalebene liegende Vorder- und/oder Rückseite des Schaftes (10) mehrere Ausnehmungen (18) aufweist.

5. Oberschenkelteil nach Anspruch 4, dadurch gekennzeichnet, daß Vorder- und/oder Rückseite des Schaftes (10) wabenartig ausgebildet ist.

6. Oberschenkelteil nach einem der Ansprüche 1 bis 5, dadurch gekennzeichnet, daß der Halsabschnitt (12) zum Schaft hin in einen sich allmählich verbreiternden Kragen (20) übergeht, der an der Unterseite eine Schulterfläche (21) zur Auflage auf das proximale Ende des bearbeiteten Femurs bildet.

7. Oberschenkelteil nach Anspruch 6, dadurch gekennzeichnet, daß die mediale Seite (14) des Schaftes (10) über eine konkave Wölbung (22) stetig in die Schulterfläche (21) übergeht.

8. Oberschenkelteil nach einem der Ansprüche 1 bis 7, dadurch gekennzeichnet, daß die laterale und/oder mediale Seite (13 bzw. 14) des Schaftes (10) senkrecht zur Frontalebene konvex gewölbt ist, vorzugsweise mit dem gleichen Radius.

9. Oberschenkelteil nach einem der Ansprüche 1 bis 8, dadurch gekennzeichnet, daß an der lateralen Seite des Halsabschnitts (12) ein Einschlagansatz (23) angeformt ist.

10. Oberschenkelteil nach Anspruch 9, dadurch gekennzeichnet, daß der Ansatz (23) eine Öffnung (24) oder einen Hinterschnitt oder dergleichen aufweist zwecks Zusammenwirkung mit einem Extraktionswerkzeug.

11. Oberschenkelteil nach einem der Ansprüche 1 bis 10, dadurch gekennzeichnet, daß die Krümmung von lateraler und medialer Schaftseite (13, 14) den gleichen Radius hat.

**Revendications**

1. Partie fémorale, d'une seule pièce, d'une endoprothèse pour articulation coxo-fémorale, comportant une tête (11), une tige (10), qui présente, dans le plan frontal, des côtés à courbure concave ou convexe, qui va en se rétrécissant de façon continue vers le bas dans ledit plan frontal, et qui peut être ancrée dans le fémur avec un ciment pour os, ainsi qu'un élément de col (12) interposé entre la tige (10) et la tête (11), caractérisée par le fait que le côté médial (14) de la tige (10), s'étendant dans la direction longitudinale de celle-ci, présente une courbure convexe, et son côté latéral (13), s'étendant dans la direction longitudinale de la tige (10), une courbure concave, la courbure étant choisie de manière que la région moyenne de la tige (10) repose, en position d'implantation, contre la corticale médiale du fémur, tandis que la région extrême inférieure (25) de ladite tige (10) est conformée de manière à prendre appui, en position d'implantation, contre la corticale latérale.

2. Partie fémorale selon la revendication 1, caractérisée par le fait que la région extrême inférieure présente une surface latérale d'appui (25), qui est à peu près parallèle à la paroi du canal médullaire au voisinage de la corticale latérale.

3. Partie fémorale selon la revendication 1 ou 2, caractérisée par le fait que la largeur de la tige (10) dans le plan frontal est plus grande, dans la région supérieure et moyenne, qui perpendiculairement à celui-ci, de préférence approximativement une fois et demie à deux fois plus grande.

4. Partie fémorale selon l'une des revendications 1 à 3, caractérisée par le fait que la face avant et/ou la face arrière de la tige (10), qui sont situées dans le plan frontal, présentent plusieurs évidements (18).

5. Partie fémorale selon la revendication 4, caractérisée par le fait que la face avant et/ou la face arrière de la tige (10) sont réalisées en nid d'abeilles.

6. Partie fémorale selon l'une quelconque des revendications 1 à 5, caractérisée par le fait que l'élément de col (12) est raccordé à la tige par un col (20), qui va en s'élargissant progressivement, et qui forme, sur la face inférieure, une surface d'épaulement (21) pour l'appui sur l'extrémité proximale du fémur à traiter.

7. Partie fémorale selon la revendication 6, caractérisée par le fait que le côté médial (14) de la tige (10) se raccorde de façon continue à la surface d'épaulement (21) par une voussure concave (22).

8. Partie fémorale selon l'une des revendications 1 à 7, caractérisée par le fait que le côté latéral et/ou médial (13 ou 14) de la tige (10) présente un bombement convexe perpendiculairement au plan frontal, de préférence de même rayon.

9. Partie fémorale selon l'une des revendications 1 à 8, caractérisée par le fait qu'un appendice pour l'enforcement par battage (23) est formé sur le côté latéral de l'élément de col (12).

10. Partie fémorale selon la revendication 9, caractérisée par le fait que l'appendice (23) présente une ouverture (24) ou une découpe, ou un élément analogue, pour coopérer avec un outil d'extraction.

11. Partie fémorale selon l'une des revendications 1 à 10, caractérisée par le fait que la courbure des côtés latéral et médial (13, 14) de la tige, a le même rayon.

## Claims

1. A one-piece femoral portion of a hip joint endoprosthesis comprising a head (11), a shank (10) having in the frontal plane respectively concavely or convexly curved sides, said shank constantly tapering downwardly in the frontal plane and adapted to be anchored in the femur with bone cement, and a neck portion (12) arranged between the shank (10) and the head (11), characterized in that the medial side (14) of the shank (10) extending longitudinally of the shank is convexly curved and the lateral side (13) extending longitudinally of the shank is concavely curved, with the curvature being selected such that the medium region of the shank (10) in the implanted condition rests against the medial corticalis of the femur with the lower end region (25) of the shank (10) being formed in such a manner that it is supported at the lateral corticalis in the implanted condition.

2. A femoral portion according to claim 1, characterized in that the lower end region has a lateral abutment surface (25) extending approximately in parallel with the wall of the medullary canal in the region of the lateral corticalis.

3. A femoral portion according to claim 1 or 2, characterized in that the width of the shank (10) in the frontal plane is larger in the upper and medium regions thereof than perpendicularly thereto, preferably, approximately one and a half to twice as large.

4. A femoral portion according to any one of the claims 1 to 3, characterized in that the front and/or rear side of the shank lying in the frontal plane is provided with several recesses (18).

5. A femoral portion according to claim 4, characterized in that the front and/or rear side of the shank (10) are honeycomb-shaped.

6. A femoral portion according to any one of the claims 1 to 5, characterized in that the neck portion (12) towards the shank merges with a gradually enlarging collar (20) said collar forming at the underside thereof a shoulder surface (21) for abutment on the proximal end of the processed femur.

7. A femoral portion according to claim 6, characterized in that the medial side (14) of the shank (10) steadily merges with the shoulder surface (21) via a concave curvature (22).

8. A femoral portion according to any one of the claims 1 to 7, characterized in that the lateral and/or medial side (13 and 14, respectively) of the shank (10) is convexly curved perpendicularly to the frontal plane, preferably at the same radius.

9. A femoral portion according to any one of the claims 1 to 8, characterized in that a drive-in extension (23) is formed integrally at the lateral side of the neck portion (12).

10. A femoral portion according to claim 9, characterized in that the extension (23) has an opening (24) or an undercut or the like for cooperation with an extraction tool.

11. A femoral portion according to any one of the claims 1 to 10, characterized in that the curvatures of the lateral and medial sides (13, 14) of the shank have one and the same radius.

# FIG.1

# FIG.2

# FIG.3